(19) Europäisches Patentamt

European Patent Office

Office européen des brevets

(11) **EP 1 430 830 A1**

(12) **EUROPEAN PATENT APPLICATION**

(43) Date of publication:
**23.06.2004 Bulletin 2004/26**

(21) Application number: **03078836.8**

(22) Date of filing: **08.12.2003**

(51) Int Cl.⁷: **A61B 5/00**, A61B 6/00, G06T 7/00, G06T 7/20, G06F 19/00

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **20.12.2002 US 327505**

(71) Applicant: **EASTMAN KODAK COMPANY**
**Rochester, New York 14650 (US)**

(72) Inventors:
• **Spoonhower, John P.**
**Rochester New York 14650-2201 (US)**
• **Jones, Emily M.**
**Rochester New York 14650-2201 (US)**
• **Stephany, Thomas M.**
**Rochester New York 14650-2201 (US)**
• **Squilla, John R.**
**Rochester New York 14650-2201 (US)**
• **Boland, John T.**
**Rochester New York 14650-2201 (US)**
• **Russell, Steven T.**
**Rochester New York 14650-2201 (US)**

(74) Representative: **Haile, Helen Cynthia et al**
**Kodak Limited,**
**Patents Department (W92-3A),**
**Headstone Drive**
**Harrow, Middlesex HA1 4TY (GB)**

(54) **Intra-oral imaging system and method for dimensional measurement below the gumline**

(57) A non-invasive method for evaluating the degree of healing around a prosthetic device or implant that is obscured by tissue employs a source of imaging radiation that illuminates an area of bone or tissue around the prosthetic device or implant. The method comprises obtaining first and second images representing the bone or tissue taken at different times; determining reference points identifying similar image positions in the first and second images; registering the first and second images by utilizing the reference points, thereby producing first and second registered images; utilizing the registered images in a subtractive process to determine the differences between the first and second images; and utilizing the differences between the first and second images to generate measurements of bone or tissue growth over time around the site of the prosthetic device or implant, thereby measuring the extent of healing around the prosthetic device or implant.

*FIG. 5*

EP 1 430 830 A1

## Description

The invention relates generally to the field of dental imaging, and in particular to the use of subtractive radiography to measure changes in human tissues and bone over time.

The use and application of dental implants is well known in the art. These types of procedures are generally utilized either where typical dental crowns are not applicable or where the use of dental bridgework is difficult or otherwise undesirable to the patient. Additionally, situations such as accidents or disease often produce damage to the teeth or underlying bone structure such that applications of typical dentistry procedures are impossible.

In a typical dental practice wherein a dentist deems a dental implant to be necessary for a patient, a typical procedure using the current state of the art may be described, as follows. For example, the replacement of a missing number 20 second premolar bicuspid of the lower jaw or mandible would involve first taking an x-ray of the area of the future surgical activity to reveal bone condition and the amount of space available to receive an implant. Upon evaluation of the x-ray, and all indications being positive, the area would be anesthetized, or the patient rendered unconscious. Next, an incision would be made through the soft tissue in the space between the good teeth where the implant is to be placed. After the incision that reveals the underlying jawbone, the soft tissue would be retracted and rinsed to prepare for the next step. A dental drilling burr would then be used to establish a point of entry for the implant in the form of a first indent into the surface of the jawbone. An approximately 2mm drill would then be utilized to make the first preparatory hole into the jawbone, with depth being established at this step. Using a 2mm depth probe, the hole is alternately drilled and measured until the desired depth is reached to receive the implant.

Additionally, this procedure "sounds" the bone in an effort to establish the overall integrity of the hole and to establish that no perforations have occurred. Next, an approximately 3mm drill or dental burr is utilized to widen the drilled hole and is again alternately checked with a 3mm probe. Again, all indications being positive to the dentist, a guide pin is inserted into the hole to check the overall integrity of the prepared site and its proper alignment to the existing teeth. Next, the dental implant, which has a round shape and has both internal and external threads, is made ready for insertion. The implant is next carefully screwed into the prepared hole using copious amounts of irrigation to prevent overheating the underlying bone. The implant is then checked for proper depth and verified to be flush with the jawbone. After all this complete, the soft tissue surrounding the site is sutured closed, thus burying the implant. The sutures are removed about ten days after surgery and the implant is left undisturbed for about six months for solidification.

After the six months, another visit is required to again incise the site to place within the implant a healing abutment or temporary crown which trains the gum tissue to grow around the future prosthesis in a collar-like fashion. The temporary crown is left in place for about eight weeks. Once the tissues are in satisfactory condition, an impression is taken with the help of transfer pins inserted into the implant. These pins transfer position of the implant to the rest of the teeth by the holes left in the impression. This impression permits the creation of custom gluing abutments from metal components which are screwed and torque pre-loaded into the implant. A permanent crown is then cemented into the abutment which also fits into the pre-grown gum tissue. This completes the implant procedure for the implant, which now only requires minor healing.

As described, it is evident that this procedure is extremely time consuming, painful for the patient, and inconvenient for both the dentist and patient. Importantly, many measurements have to be taken which are obviously inexact and subject to interpretation by the dental practitioner. These measurements are needed to measure and track changes in the underlying extent of healing of both hard (bone) and soft tissue structure to assess the readiness for subsequent procedures. Moreover, such measurements may involve disturbing the tissue, not to speak of the patient, in order to see the area and/or to position the necessary measuring probes and tools.

U.S. Patent Nos. 5,664,574 and 6,058,324, which both issued in the name of B. Chance, disclose methods and systems for in vivo, non-invasive examination of a subject using non-ionizing radiation such as near Infra-Red radiation. Additionally, U.S. Patent No. 6,419,484 B1, which issued in the name of DaSilva et al., discloses a dental drill comprising one or more single mode fibers that are used to image in the vicinity of the drill tip.

Notwithstanding these imaging approaches taken in the prior art, there is a need for an intra-oral imaging methodology which is capable of removing the subjectivity and interpretation inherent in the use of such procedures. More specifically, there is a need for an intra-oral imaging system permitting measurements below the gumline and software such as subtractive radiography to reduce the error and improve the accuracy of such procedures. Such technical solutions to these problems would provide system and methodology to measure and track changes in the underlying extent of healing of both hard (bone) and soft tissue structure to better assess the readiness for subsequent procedures.

It is an object of the invention to facilitate the measurement of teeth and /or bone dimensions in order to assist dental practitioners in the proper preparation of a prosthesis.

The present invention is directed to overcoming one or more of the problems set forth above. Briefly summarized, according to one aspect of the present invention, a non-invasive method for evaluating the degree of healing around a prosthetic device or implant that is obscured by tissue employs a source of imaging radiation that illuminates an area of bone or tissue around the prosthetic device or implant. The method comprises obtaining first and second images representing the bone or tissue taken at different times; determining reference points identifying similar image positions in the first and second images; registering the first and second images by utilizing the reference points, thereby producing first and second registered images; utilizing the registered images in a subtractive process to determine the differences between the first and second images; and utilizing the differences between the first and second images to generate measurements of bone or tissue growth over time around the site of the prosthetic device or implant, thereby measuring the extent of healing around the prosthetic device or implant.

From another perspective, the invention comprises a non-invasive system for measuring the dimensions of intra-oral tissue or bone growth around an prosthesis or implant situated below the gum line. This system includes a source of near infrared light; an intra-oral imaging device for illuminating the prosthesis or implant below the gumline with the near infrared light, wherein the imaging device also captures one or more images of the prosthesis or implant in the near infrared spectral region; and a processor using the captured images to generate reference points for registering the images of the prosthesis or implant below the gumline, said processor using the registered images to generate measurements of the dimensions of the tissue or bone growth over time around the prosthesis or implant. The measurements produced by the system may be used to generate a dental prosthesis, or to evaluate the extent of healing around the prosthesis or implant.

The advantage of the invention is that it enables more rapid determination of critical dimensions for practitioners, and in particular without the need, as is the current practice, for surgery to expose the underlying bone/tooth structure to enable micrometer determination of these critical dimensions. Furthermore, the advantage of the invention is that it improves upon the use of a subtractive radiography system to enhance the application of dental implants and provides means with which to more accurately and cost effectively measure parameters that dental practitioners need to assess the progress of such procedures.

These and other aspects, objects, features and advantages of the present invention will be more clearly understood and appreciated from a review of the following detailed description of the preferred embodiments and appended claims, and by reference to the accompanying drawings.

FIG. 1 is a perspective diagram of a computer system for implementing the present invention.
FIG. 2 is a pictorial illustration of an intra-oral camera that is connectable with the computer system shown in Figure 1.
FIG. 3 is a drawing of a human lower jaw or mandible detailing a missing premolar bicuspid.
FIG. 4 is a drawing of a human lower jaw or mandible detailing an implanted replacement premolar bicuspid.
FIG. 5 is a pictorial diagram of a fiber optic bundle system emitting near infra-red light into the area of the implant as shown in Figure 4, and a corresponding fiber optic bundle receiving near infra-red light from the emitter.
FIG. 6 is a pictorial diagram of an x-ray source emitting x-ray radiation into the area of the implant as shown in Figure 4, and a dental x ray film for receiving the radiation from the emitter.
FIG. 7 is a block diagram of the various stages of the multi-view registration method that is used in the subtractive radiography process according to the invention.

Because dental imaging devices employing electronic emitters and sensors and systems employing subtractive radiography are well known, the present description will be directed in particular to elements forming part of, or cooperating more directly with, system and method in accordance with the present invention. Elements not specifically shown or described herein may be selected from those known in the art. Certain aspects of the embodiments to be described may be provided in software. Given the system and method as shown and described according to the invention in the following materials, software not specifically shown, described or suggested herein that is useful for implementation of the invention is conventional and within the ordinary skill in such arts.

Still further, as used herein, the computer program may be stored in a computer readable storage medium, which may comprise, for example; magnetic storage media such as a magnetic disk (such as a hard drive or a floppy disk) or magnetic tape; optical storage media such as an optical disc, optical tape, or machine readable bar code; solid state electronic storage devices such as random access memory (RAM), or read only memory (ROM); or any other physical device or medium employed to store a computer program.

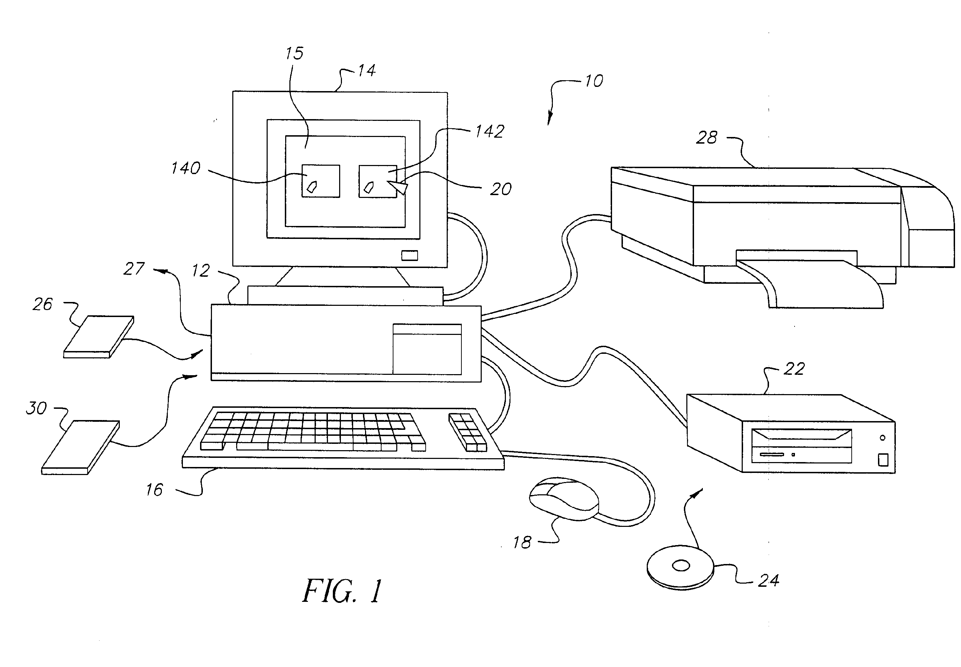

Referring to Fig. 1, there is illustrated a computer system 10 for implementing the present invention. Although the computer system 10 is shown for the purpose of illustrating a preferred embodiment, the present invention is not limited to the computer system 10 shown, but may be used on any electronic processing system. The computer system 10 includes a microprocessor-based unit 12 for receiving and processing software programs and for performing other processing functions. A display 14 is electrically connected to the microprocessor-based unit 12 for displaying user-related information associated with the software, e.g., by means of a graphical user interface (GUI) 15. A keyboard 16 is also connected to the microprocessor based unit 12 for permitting a user to input information to the software. As an alternative to using the keyboard 16 for input, a mouse 18 may be used for moving a selector (cursor) 20 on the display 14 and for selecting an item on which the selector 20 overlays, as is well known in the art.

A compact disk-read only memory (CD-ROM) 22 is connected to the microprocessor based unit 12 for receiving software programs and for providing a means of inputting the software programs and other information to the microprocessor based unit 12 via a compact disk 24, which typically includes a software program. In addition, a floppy disk 26 may also include a software program, and is inserted into the microprocessor-based unit 12 for inputting the software program. Still further, the microprocessor-based unit 12 may be programmed, as is well known in the art, for storing the software program internally. The microprocessor-based unit 12 may also have a network connection 27, such as a telephone line, to an external network such as a local area network or the Internet. Accordingly, the software program may be received over the network, perhaps after authorizing a payment to a network site. A printer 28 is connected to the microprocessor-based unit 12 for printing a hardcopy of the output of the computer system 10.

Images may also be displayed as part of the graphical user interface 15 on the display 14 via a personal computer card (PC card) 30, such as, as it was formerly known, a PCMCIA card (based on the specifications of the Personal Computer Memory Card International Association) which contains digitized images electronically embodied in the card 30. The PC card 30 is ultimately inserted into the microprocessor based unit 12 for permitting visual display of the image on the display 14. Images may also be input via the compact disk 24, the floppy disk 26, or the network connection 27. Any images stored in the PC card 30, the floppy disk 26 or the compact disk 24, or input through the network connection 27, may have been obtained from a variety of sources, such as an intra-oral camera or an x-ray image scanner, which scans x-ray films, e.g., dental films, and provides scan signals corresponding to the film images.

Near-infrared (NIR, i.e., 700 - 1000nm) light has been shown to penetrate soft tissue and allow for a method of imaging inside soft tissue structures. Such techniques as optical coherence tomography (OCT) and NIR confocal microscopy have shown the ability to image subsurface structure with penetration depths of the order of millimeters. Furthermore, the ability of light to penetrate hard tissues such as bone or teeth is significantly different than the penetration of soft tissue so that the interface between hard and soft tissue is evident using NIR imaging technologies.

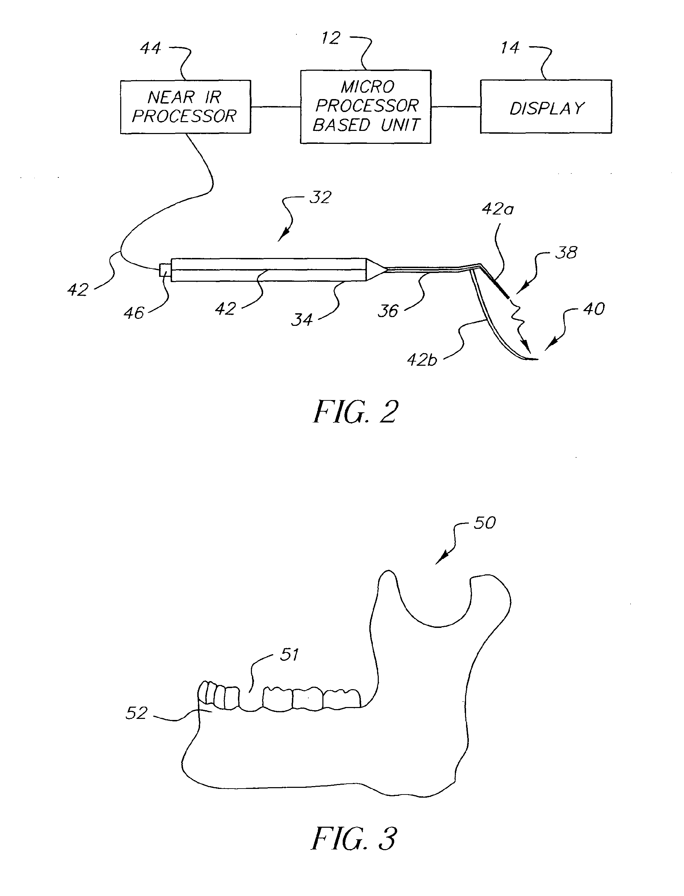

An intra-oral camera for emitting near infra-red radiation is shown in Figure 2 as a hand held fiber optic imaging device 32. The device comprises a hand-held portion 34 by which the operator can manually manipulate the device, and a probe portion 36 that can easily be inserted into a patient's mouth. The device can be designed so that the probe is manipulated robotically or remotely, in which case a handle for an operator is unnecessary. The probe portion is comprised of a fiber bundle 42 having two optical fiber parts: the first part 42a terminating at a distal end thereof in an emitter tip 38 and a second portion 42b terminating at a distal end thereof in a pickup tip 40, where the terminating points of the respective tips are separated by an operative spacing d. The shape of the probe portion 36 is designed to comfortably access as much of the oral cavity as possible, and in particular the optical parts 42a and 42b are curved such that a tooth, tissue or bone area may be disposed, as will be shown, within the operative spacing d.

The imaging device 32 supports the fiber bundle 42, which couples near infra-red light from the generator stage of a near IR processor 44 to the emitter tip 38, and from the pickup tip 40 to the receiver stage of the near IR processor 44. Near IR light is emitted from the emitter tip 38 through the distal end of the fiber part 42a, and near IR light is collected by the pickup tip 40 through the distal end of the fiber part 42b. The probe portion 36 may be detachable from the hand held portion 34 for ease of use, cleaning or disposal. A fiber connector 46 may be connected to the end of the hand held portion 34. The near IR processor 44, which generates and receives the near IR radiation, is connected to the microprocessor- based unit 12 of Figure 1, which processes the resultant signals and produces an analysis signal for display on the display 14.

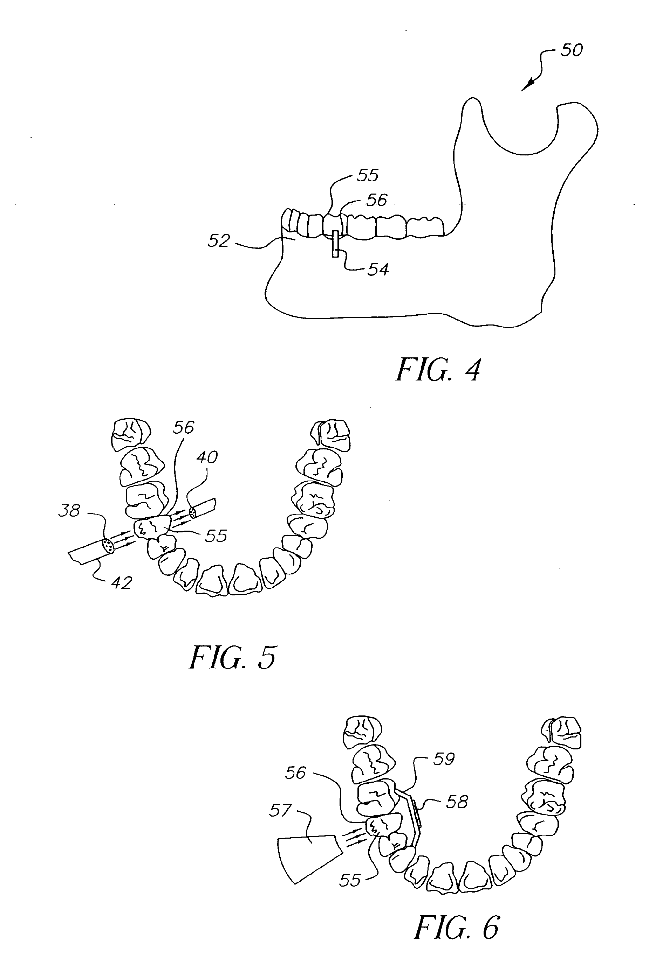

Referring now to Fig. 3, detailed is a rendition of the human lower jaw or mandible 50 before the dental restorative procedure is effected, with a missing number 20 second premolar bicuspid 51 of the lower jaw or mandible 50. The gumline 52 shown detailed in crosshatch shows the area below which detailed measurements are desired. Figure 4 shows the same lower jaw or mandible 50 after an implant 54 is inserted into the underlying jawbone and a crown 55 is cemented onto the implant 54. Figure 4 also shows an area of tissue/bone growth 56 around the implant post 54. The dimensions, or other evidence, of the region of growth 56 is determined according to the disclosed process, as will be explained.

Referring to Figure 5, the near infra-red probe light emitted from the distal end of the emitting fiber part 42a is directed from the emitter tip 38 of the probe portion 36 at or onto the hard or soft tissue 56 at the appropriate location. After transmitting through the hard or soft tissue 56, the near infra-red light is collected by the pickup tip 40 at the distal end of the pickup fiber part 42b and conducted back through the probe 56 to the near IR processor 44. The signals from the near IR processor 44 may then be transferred to the computer system 10 through a direct connection, through removable media, through a network connection, such as a local are network (e.g., Ethernet) or a public network (e.g., the Internet), or by any other convenient means.

Figure 6 shows another embodiment of the imaging portion of the invention, where x-rays from a conventional x-ray source 57 are directed at or onto the hard or soft tissue 56 at the appropriate location. After transmitting through the hard or soft tissue 56, the x-ray radiation exposes an x-ray film 58 supported in place by a film holder 59. The holder 59 may be the conventional type of film holder, such a bite wing, for holding a film in a designated place within the intra-oral cavity. After photographic development, the developed film 58 may be scanned, or otherwise read, and the resultant signals are transferred to the computer system 10 through a direct connection, through removable media, through a network connection, such as a local are network (e.g., Ethernet) or a public network (e.g., the Internet), or by any other convenient means.

An x-ray examination system can be either a traditional film system where different films can be optimized for detection of hard and soft tissue differences, or a digitally based system. In a digital system a solid state digital radiography sensor, (DR) sensor is used (in place of the film holder 59) where electronic means can be used to change contrast or contrast filters can be inserted before the DR sensor to achieve the same result, effectively repositioning the density curve of the resultant image.

Figure 6 also serves to illustrate a variation of the near infra-red system shown in Figure 5, wherein the component 57 may instead be thought of as the distal end of the emitting fiber part 42a, from which near infra-red light is directed from the emitter tip 38 of the probe portion 36 at or onto the hard or soft tissue 56 at the appropriate location. After transmitting through the hard or soft tissue 56, the near infra-red light is then imaged upon the film 58, which may be a specialized film adapted for the near infra-red emission with appropriate spectral sensitization of the emulsion.

In dealing with images taken over time under, e.g., different exposure conditions, several problems may occur. One problem is dealing with the differences and inconsistencies between images over time, which do not relate to the tissue or bone growth, but that may be sufficient to mask the changes that we are looking for, i.e., tissue and/or bone growth. For film, such differences could be non-image related density differences. Density differences in dental x-rays taken at different times can be due to a number of sources including variations in the film, illumination differences, incidence angle of the x-ray source, and exposure differences. The use of a density target such as described in commonly-assigned, co-pending U.S. Patent Application Serial No. (our Docket 85295), entitled "Incorporation of a Density Target with Dental Films to Facilitate Subtractive Radiography" and filed on even date herewith in the names of J. Squilla, J. Boland and J. Spoonhower, and which is incorporated herein by reference, can correct for these issues by allowing the places of predetermined minimum and maximum exposure values to be positively identified and measured. The density measurements from the minimum and maximum points are used by software to correct and match the dynamic range of the x-rays taken at different times.

(More specifically, co-pending U.S. Patent Application Serial No. (our Docket 85295) discloses a method and system for equalizing non-diagnostic differences that occur in two or more radiographic images taken of the same object at different times. The described technique utilizes an x-ray radiation source that generates a beam of radiation and an image receiver (e.g., a film) positioned to receive radiation, from the radiation source, that interacts with the object, whereby image data of the object is captured by the film receiver. The method includes the steps of interposing a graded density target in the path of the beam of radiation between the source and the image receiver such that the target is imaged upon the image receiver together with the object; using the radiation source and the receiver to capture two or more images of the object at different times; generating measurements of the targets in each of the captured images; and using the measurements to equalize the image data of the radiographic images, thereby generating two or more equalized images that have been processed to equalize the non-diagnostic differences between the images.)

Another problem would be registration of the two images, in particular since the images are captured via a non-precise hand held imaging device. In other words, since the invention is useful in a subtractive radiography process where change detection is used to identify areas of differences among images of the same region that were collected at different times, registration of the images is a prerequisite for the change detection process. Registration of images taken at different times can be accomplished interactively using a process similar to that described in accordance with the cross-referenced commonly assigned copending application Serial No. 09/970,243, which is incorporated herein by reference, and in which a series of comparative views of related images are produced and presented to a user through the graphical user interface 15 presented on the display 14. More specifically, these comparative views enable user-friendly registration of the images prior to engaging in a subtractive process for isolating changes between the images. Automatic registration can be accomplished via software by detecting and recognizing unique points on the dental implant as they appear in each x-ray, using either existing points or points purposefully added to the implant for this purpose. Alternatively, automatic registration can be accomplished via software by matching the shape, or outline, of the dental implant in each x-ray.

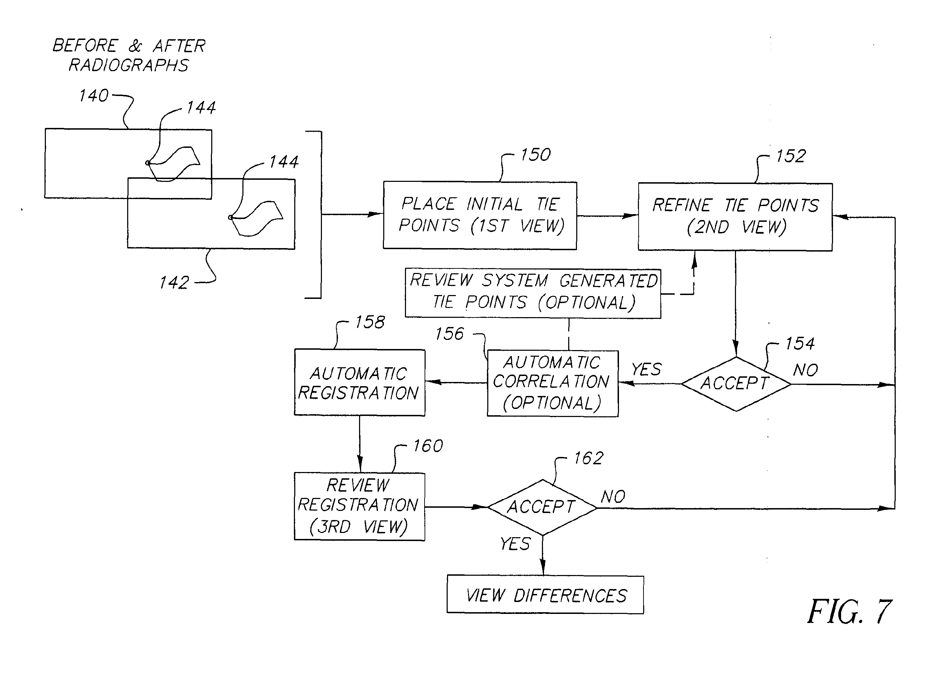

As also described in commonly assigned copending application Serial No. 09/970,243, an automated method for placing reference points in a radiography application is shown in its several stages in Figure 7 for the two images 140 and 142, which represent before and after images of an oral object, such as a tooth ("before" and "after" is meant to represent a time sequence that would reveal, e.g., changes in the tooth, bone or tissue structure caused by a cavity or disease, or by the aforementioned implant process). The images 140 and 142 are processed by the computer system 10 and presented to a user via the graphical user interface 15, whereupon the user manipulates and places potential tie points 144 on the images 140 and 142 by using the mouse 18 or the keyboard 16. For example, the selector (cursor) 20 can be used to locate the potential tie points, as also represented in the view shown in Figure 1, where the two images are shown side by side. This is done by means of conventional software in the placement stage 150 that records coordinates for the selected tie points, and which prompts the user to switch between images in a predetermined manner, i.e., once a first point has been placed in the left image 140, the user must place a second point in the right image 142, and so on through the other reference points. At least three reference points are selected for the subsequent registration process, and more points are preferred for a more accurate and robust registration.

In the refinement stage 512, more detailed views are processed by conventional software that isolates an area around each of the potential tie points and magnifies and presents each area in sequence to the user through the graphical user interface 15. After the tie points are refined to the liking of the user, acceptance is signaled by an acceptance decision 154 through manipulation of the mouse 18 or the keyboard 16 (if, for any reason, the results are unacceptable, the process is returned to the refinement stage 152 until the result is acceptable). The result is a set of refined tie points that are suitable for the registration process.

In other words, the placement stage 150 presents a "zoomed out" view of the full extent of the first and second images side by side in a first graphical user interface view in order to allow a user to place potential reference points in their approximate locations on each image, all the while maintaining context for the user. Thereafter, the refinement stage 152 presents a "zoomed in" view of an area of the first and second images around each of the potential tie points in a second graphical user interface view to allow the user to refine the placement of the potential reference points, thereby enabling the generation of refined reference points suitable for registration, wherein the reference points identify similar image positions in separate images of substantially the same bodily object.

Once accepted, the refined tie points can be used in conjunction with optional automatically correlated points in the correlation stage 156. These optional points may then be reviewed by the user. In the auto registration stage 158, a polynomial function is generated to relate the tie points. In its simplest form, the polynomial (alignment equation) is of the form

$$X=a_1+a_2X'+a_3Y'$$

with only three constants (and a similar equation for Y). Hence, locating three reference (tie) points that are common to two sequential images allows one to be rotated and stretched (warped) to align with the other. (See pages 201 - 208 on Alignment in The Image Processing Handbook, Second Edition, by John C. Russ, CRC Press, 1995). Typically, more tie points are involved in the registration process. For instance, in commonly-assigned U.S. Patent No. 6,163,620 (entitled "Automatic Process for Detecting Changes Between Two Images"), which is incorporated herein by reference, between five and one hundred tie points are used. The polynomial function is then used in the auto registration stage 158 to warp the right image 142 to the left image 140 (or vice versa). Once registration is completed, the results are aligned side by side for review in the registration review stage 160. Known alignment techniques may be employed to render the left and right images for this view with the same zoom level and image centering. (cf., The Image Processing Handbook). Given the correlation between the images at this point, cursor placement in the left image can be mimicked by cursor placement at the same place in the right image. Likewise, a "zoom-in" on the left image can exactly matched by a corresponding "zoom-in" of the right image. If the user deems the registration adequate, acceptance is signaled by the acceptance decision 162 through manipulation of the mouse 18 or the keyboard 16; otherwise, the process is returned to the refinement stage 152 and repeated in an iterative manner until the registration results are acceptable to the user.

This invention is intended to enable an accurate registration of the two images prior to a subtractive radiography process. In a subtractive process of this type, subtracting one image from another effectively removes from the difference image all features that do not change, while highlighting or otherwise denoting those that do. Details of such a subtractive process, though not used in connection with radiography, are disclosed in the aforementioned U. S. Patent No. 6,163,620, which is incorporated herein by reference. In a dental environment, this process can be used to isolate various types of temporal changes between radiographs of the same object taken at different times, e.g., to isolate bone loss due to periodontal disease (by looking under the gum line). It should be understood, however, that the registration review stage 160 is capable of producing a visual "differencing" effect (i.e., flickering) between the two images that may be sufficient in some cases to indicate the temporal change between the two images.

The techniques described herein relates in general to a non-invasive method for measuring the dimensions of a bodily object obscured by tissue in order to measure changes in the bodily object over time. While described in relation to a prosthesis implanted in a dental procedure and operation, it should be understood that the method is not to be seen as being limited in this way and would apply to any artificial device used to replace, reinforce, support or otherwise assist a missing, weakened, damaged or similarly affected body part, such as a missing, weakened or damaged limb.

**Claims**

1. A non-invasive method for evaluating the degree of healing around a prosthetic device or implant that is obscured by tissue, said method comprising the steps of:

   providing a source of imaging radiation;
   illuminating an area of bone or tissue around the prosthetic device or implant with the imaging radiation;
   obtaining first and second images representing the bone or tissue taken at different times;
   determining reference points identifying similar image positions in the first and second images;
   registering the first and second images by utilizing the reference points, thereby producing first and second registered images;
   utilizing the registered images in a subtractive process to determine the differences between the first and second images; and
   utilizing the differences between the first and second images to generate measurements of bone or tissue growth over time around the site of the prosthetic device or implant, thereby measuring the extent of healing around the prosthetic device or implant.

2. The method as claimed in claim 1 wherein the step of determining reference points comprises the steps of:

   presenting a "zoomed out" view of the full extent of the first and second images side by side in a first graphical user interface view in order to allow a user to place potential reference points in their approximate locations on each image, all the while maintaining context for the user; and
   presenting a "zoomed in" view of an area of the first and second images around each of the potential tie points in a second graphical user interface view to allow the user to refine the placement of the potential reference points, thereby enabling the generation of refined reference points suitable for registration.

3. The method as claimed in claim 1 wherein the step of utilizing the registered images in a subtractive process provides a difference image indicative of changes between the first and second images.

4. The method as claimed in claim 1 wherein the step of registering the first and second images comprises producing a polynomial correlation between the reference points of the first and second images and warping the two images until two registered images are obtained.

5. The method as claimed in claim 1 wherein the imaging radiation is near infrared radiation.

6. The method as claimed in claim 1 wherein the imaging radiation is x-ray radiation.

7. A non-invasive method for measuring the dimensions of intra-oral tissue or bone growth around an prosthesis or implant situated below the gum line, said method comprising the steps of:

   providing a source of near infrared light;
   illuminating the prosthesis or implant below the gumline with the near infrared light;
   capturing one or more images of the prosthesis or implant in the near infrared spectral region;
   using the captured images to generate reference points for registering the images of the prosthesis or implant below the gumline; and
   using the registered images to generate measurements of the dimensions of the tissue or bone growth over time around the prosthesis or implant.

8. The method as claimed in claim 7 wherein the measurements are used to generate a dental prosthesis.

9. The method as claimed in claim 7 wherein the measurement s are used to evaluate the extent of healing around the prothesis or implant.

FIG. 1

FIG. 2

FIG. 3

FIG. 4

FIG. 5

FIG. 6

FIG. 7

European Patent Office

**EUROPEAN SEARCH REPORT**

Application Number

EP 03 07 8836

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| L,P, X | US 2003/063788 A1 (SPOONHOWER JOHN P ET AL) 3 April 2003 (2003-04-03) Cited for priority reasons. --- | 1-4,6 | A61B5/00 A61B6/00 G06T7/00 G06T7/20 G06F19/00 |
| X | LEHMANN T M ET AL: "Computer-based registration for digital subtraction in dental radiology" DENTO-MAXILLO-FACIAL RADIOLOGY, INT. ASS. DENTO-MAXILLO-FACIAL RADIOLOGY, GOTEBORG, SE, November 2000 (2000-11), pages 323-346, XP002213379 ISSN: 0250-832X | 1,3,4,6 | |
| Y | Sect.3.1.5, Sect.4 * figure 1 * --- | 5,7-9 | |
| Y | US 5 818 587 A (INABA HUMIO ET AL) 6 October 1998 (1998-10-06) * column 2, line 59 - column 3, line 6; claim 3; figure 7 * * column 6, line 6-34 * --- | 5,7-9 | |
| A | DE 43 07 411 A (MIRA GMBH) 15 September 1994 (1994-09-15) * column 3, line 25-29; figure 1A * * column 4, line 46-51 * * column 5, line 54-68 * --- | 5,7 | |
| X | REDDY MS, JEFFCOAT MK, RICHARDSON RC: "Assessment of adj flurbiprofen ther" J ORAL IMPLANT, vol. 16, no. 4, 1990, pages 272-6, XP008029193 * abstract * * page 274, left-hand column, paragraph 2 * --- | 1,3,6 | |

TECHNICAL FIELDS SEARCHED (Int.Cl.7)

A61B
G06T
G06F

-/--

The present search report has been drawn up for all claims

| Place of search | Date of completion of the search | Examiner |
|---|---|---|
| BERLIN | 30 March 2004 | Kronberger, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

12

**European Patent Office**

## EUROPEAN SEARCH REPORT

Application Number

EP 03 07 8836

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | BYRD V, MAYFIEL-DONAHOO T, REDDY MS, JEFFCOAT MK: "Semiautomated image registration for digital subtraction radiography" ORAL SURG ORAL MED ORAL PATHOL ORAL RADIOL ENDOL, vol. 85, no. 4, 1998, pages 473-8, XP008029194 * abstract * | 2 | |
| A | WENZEL A: "EFFECT OF MANUAL COMPARED WITH REFERENCE POINT SUPERIMPOSITION ON IMAGE QUALITY IN DIGITAL SUBTRACTION RADIOGRAPHY" DENTO-MAXILLO-FACIAL RADIOLOGY, INT. ASS. DENTO-MAXILLO-FACIAL RADIOLOGY, GOTEBORG, SE, vol. 18, November 1989 (1989-11), pages 145-150, XP009001206 ISSN: 0250-832X * abstract * | 2 | |

TECHNICAL FIELDS SEARCHED (Int.Cl.7)

The present search report has been drawn up for all claims

| Place of search | Date of completion of the search | Examiner |
|---|---|---|
| BERLIN | 30 March 2004 | Kronberger, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 03 07 8836

This annex lists the patent family members relating to the patent documents cited in the above-mentioned European search report.
The members are as contained in the European Patent Office EDP file on
The European Patent Office is in no way liable for these particulars which are merely given for the purpose of information.

30-03-2004

| Patent document cited in search report | Publication date | Patent family member(s) | | Publication date |
|---|---|---|---|---|
| US 2003063788 A1 | 03-04-2003 | NONE | | |
| US 5818587 A | 06-10-1998 | JP | 2879003 B2 | 05-04-1999 |
| | | JP | 9135853 A | 27-05-1997 |
| DE 4307411 A | 15-09-1994 | DE | 4307411 A1 | 15-09-1994 |
| | | WO | 9420011 A2 | 15-09-1994 |
| | | EP | 0688180 A1 | 27-12-1995 |

EPO FORM P0459

For more details about this annex : see Official Journal of the European Patent Office, No. 12/82